(19)

Europäisches
Patentamt
European
Patent Office
Office européen
des brevets

(11)  **EP 4 636 781 A1**

(12)  **EUROPEAN PATENT APPLICATION**
published in accordance with Art. 153(4) EPC

(43) Date of publication:
**22.10.2025  Bulletin 2025/43**

(21) Application number: **23901897.1**

(22) Date of filing: **08.02.2023**

(51) International Patent Classification (IPC):
**G16H 50/20** (2018.01)

(86) International application number:
**PCT/CN2023/075024**

(87) International publication number:
**WO 2024/124677 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **13.12.2022  CN 202211635867**

(71) Applicants:
• **Peking University Third Hospital
  Beijing 100191 (CN)**
• **Hangzhou Qingguo Medical Technology Co., Ltd.
  Hangzhou, Zhejiang 310000 (CN)**

(72) Inventors:
• **LI, Rong
  Beijing 100191 (CN)**
• **XU, Huiyu
  Beijing 100191 (CN)**
• **QIAO, Jie
  Beijing 100191 (CN)**
• **FENG, Guoshuang
  Beijing 100191 (CN)**
• **HAN, Yong
  Hangzhou, Zhejiang 310000 (CN)**
• **SHI, Li
  Beijing 100191 (CN)**

(74) Representative: **Keller Schneider
Patentanwaltsgesellschaft mbH
Linprunstraße 10
80335 München (DE)**

(54)  **SYSTEM AND METHOD FOR SCREENING FOR POLYCYSTIC OVARIAN SYNDROME**

(57)  The present application relates to a system for screening for polycystic ovarian syndrome (PCOS). The system comprises: a data collection module, which is used for acquiring an anti-mullerian hormone (AMH) level of a subject, collecting an upper limit of the number of days of a menstrual cycle that is actively provided by the subject, and collecting data of the BMI of the subject; and a module for calculating the probability (p) of suffering from PCOS, wherein the module is used for performing calculation on data information acquired in the data collection module, so as to calculate p of the subject suffering from PCOS. By means of the system constructed in the present application, only three parameters, i.e., an AMH, the number of days of a menstrual cycle and the BMI, are used, such that the problem of androgen examination, by means of an immunization method, being inaccurate is prevented, thereby further reducing an examination time and the examination cost, and improving the examination efficiency and accuracy. Moreover, the applicant of the present application performs research in the real world, the sample volume is large, and it is presented by means of external verification that an optimized three-index PCOS model and the previous four-index model have the same prediction effect.

EP 4 636 781 A1

**Description**

TECHNICAL FIELD

This application relates to a system and method for evaluating the probability of a subject suffering from polycystic ovary syndrome, as well as a system and method for assisting in screening polycystic ovary syndrome. The system and the method of this application can be used to evaluate the probability of a subject suffering from polycystic ovary syndrome, thereby assisting in screening whether a subject suffers from polycystic ovary syndrome, and evaluating whether the condition of a subject suffering from polycystic ovary syndrome has improved after corresponding treatment.

BACKGROUND

Polycystic ovary syndrome (PCOS) affects over 10% of women worldwide, and is one of the most common endocrine and metabolic diseases, requiring special attention to the long-term health issues of this population. Only a small proportion of PCOS patients seek medical attention due to infertility factors, while a considerable proportion of PCOS patients do not seek medical attention. Therefore, a considerable number of PCOS patients are unable to effectively manage their potential metabolic disease risk in the future. In addition, due to the unknown pathogenesis of PCOS, the commonly used diagnostic criteria internationally are also controversial.

Based on the above background, there is an urgent need for new screening standards related to the pathogenesis of PCOS in this field. In addition, current clinical practices for screening and diagnosing PCOS are not easy for general gynecologists and primary care physicians, often leading to missed diagnosis.

SUMMARY OF THE INVENTION

Based on the problems in the prior art, the applicant provided a system for diagnosing polycystic ovary syndrome in the previous patent CN 113035354 B, which comprises: a data collection module for obtaining anti-Mullerian hormone (AMH) level in a subject, collecting upper limit of the number of menstrual cycle days voluntarily provided by the subject, and collecting BMI of the subject, and obtaining androstenedione (A4) level of the subject; and a module for calculating a probability of suffering from polycystic ovary syndrome, which is used to calculate the above data information obtained from the data collection module, so as to calculate the probability (p) of a subject suffering from polycystic ovary syndrome.

Due to the poor accuracy of the immunoassay-based chemiluminescence method for detecting androstenedione (A4) in clinical practice, the correlation $r^2$ between immunoassay-based A4 and gold standard mass spectrometry in women is only 0.285 (Zhang X, Xu H, Feng G, et al. Clin Chim Acta doi: 10.1016/j.cca.2022.11.025) PubMed PMID: 36450312.), equivalent to in women, A4 of the immunoassay-based chemiluminescence method can only explain 28.5% of A4 of the mass spectrometry. In this application, the inventors attempt to establish a system and method for screening and predicting PCOS by using fewer indicators, thereby further reducing testing time and cost, and improving testing efficiency and accuracy.

Particularly, this application involves the following contents:

1. A system for screening polycystic ovary syndrome, comprising:

a data collection module for obtaining anti-Mullerian hormone (AMH) level in a subject, collecting upper limit of the number of menstrual cycle days voluntarily provided by the subject, and collecting BMI of the subject; and
a module for calculating a probability of suffering from polycystic ovary syndrome, which is used to calculate the above data information obtained from the data collection module, so as to calculate a probability (p) of a subject suffering from polycystic ovary syndrome.

2. The system according to item 1, further comprising:
a grouping module for pre-storing default grouping parameters for polycystic ovary syndrome, grouping the calculated probability (p) of a subject suffering from polycystic ovary syndrome based on the grouping parameters, thereby grouping the risk of a subject suffering from polycystic ovary syndrome.

3. The system according to item 1 or 2, wherein
in the module for calculating a probability of suffering from polycystic ovary syndrome, the probability (p) of a subject suffering from polycystic ovary syndrome is calculated by converting data of the anti-Mullerian hormone (AMH) level, upper limit of the number of menstrual cycle days, and BMI of the subject into polytomous variables.

4. The system according to item 1 or 2, wherein
the anti-Mullerian hormone (AMH) level refers to a concentration of AMH in venous blood of a female subject on any day of the menstrual cycle.

5. The system according to item 1 or 2, wherein

in the module for calculating a probability of suffering from polycystic ovary syndrome, the levels of anti-Mullerian hormone (AMH) are converted into five-categorical variables, that is to say,
the AMH levels are divided into the following five groups: the subjects' AMH levels are less than 2.5 ng/ml, the subjects' AMH levels are 2.5 ng/ml or more but less than 5 ng/ml, the subjects' AMH levels are 5 ng/ml or more but less than 7.5 ng/ml, the subjects' AMH levels are 7.5 ng/ml or more but less than 10 ng/ml, and the subjects' AMH levels are 10 ng/ml or more.

6. The system according to item 1 or 2, wherein

in the module for calculating a probability of suffering from polycystic ovary syndrome, the upper limits of the number of menstrual cycle days of the subjects are converted into five-categorical variables, that is to say,
the upper limits of the number of menstrual cycle days of the subjects are divided into the following five groups: the upper limits of the number of menstrual cycle days of the subjects are less than 35 days, the upper limits of the number of menstrual cycle days of the subjects are 35 days or more but less than 45 days, the upper limits of the number of menstrual cycle days of the subjects are 45 days or more but less than 60 days, the upper limits of the number of menstrual cycle days of the subjects are 60 days or more but less than 90 days, and the upper limits of the number of menstrual cycle days of the subjects are 90 days or more.

7. The system according to item 1 or 2, wherein

in the module for calculating a probability of suffering from polycystic ovary syndrome, the BMIs of the subjects are converted into four-categorical variables, that is to say,
the BMIs of the subjects are divided into the following four groups: BMIs of the subjects are less than 18.5, BMIs of the subjects are 18.5 or more but less than 24, BMIs of the subjects are 24 or more but less than 28, and BMIs of the subjects are 28 or more.

8. The system according to item 1 or 2, wherein
in the module for calculating a probability of suffering from polycystic ovary syndrome, a formula for calculating a probability (p) of suffering from polycystic ovary syndrome is pre-stored, and the formula is obtained by fitting the polytomous variables converted from the data of the anti-Mullerian hormone (AMH) level, upper limit of the number of menstrual cycle days, and BMI of the subject in the existing database.
9. The system according to item 8, wherein
the above formula is the following Formula I:

$$p=1/[1+e^{-(i+a*AMH+b*\text{upper limit of the number of menstrual cycle days}+c*BMI)}] \quad (\text{Formula I})$$

wherein p is the calculated probability of a subject suffering from polycystic ovary syndrome, and a, b, c and i are unitless parameters;
in the module for calculating a probability of suffering from polycystic ovary syndrome, the values of a, b and c are obtained based on the anti-Mullerian hormone (AMH) level, upper limit of the number of menstrual cycle days, and BMI of the subject and are plugged into Formula I for calculation,
and in the calculation, the value of AMH, the upper limit of the number of menstrual cycle days, or BMI is set as 0 or 1.

10. The system according to item 9, wherein

i is any value selected from -4.255406 to -3.56205;
when the subject's AMH level is less than 2.5 ng/ml, the AMH value is set as 0;
when the subject's AMH level is 2.5 ng/ml or more but less than 5 ng/ml, the AMH value is set as 1, and a is any value selected from 0.3982787 to 0.846642;
when the subject's AMH level is 5 ng/ml or more but less than 7.5 ng/ml, the AMH value is set as 1, and a is any value selected from 1.3775495 to 1.8533111;
when the subject's AMH level is 7.5 ng/ml or more but less than 10 ng/ml, the AMH value is set as 1, and a is any value selected from 2.0095086 to 2.5992375;
when the subject's AMH level is 10 ng/ml or more, the AMH value is set as 1, and a is any value selected from 2.4552789 to 3.0324161;

when the upper limit of the number of menstrual cycle days of the subject is less than 35 days, the value of the upper limit of the number of menstrual cycle days is set as 0;

when the upper limit of the number of menstrual cycle days of the subject is 35 days or more but less than 45 days, the value of the upper limit of the number of menstrual cycle days is set as 1, and b is any value selected from 1.1836336 to 1.6602398;

when the upper limit of the number of menstrual cycle days of the subject is 45 days or more but less than 60 days, the value of the upper limit of the menstrual cycle days is set as 1, and b is any value selected from 1.6734536 to 2.1734828;

when the upper limit of the number of menstrual cycle days of the subject is 60 days or more but less than 90 days, the value of the upper limit of the number of menstrual cycle days is set as 1, and b is any value selected from 1.7119305 to 2.4218832;

when the upper limit of the number of menstrual cycle days of the subject is 90 days or more, the value of the upper limit of the number of menstrual cycle days is set as 1, and b is any value selected from 2.0049859 to 2.682179;

when the subject's BMI is less than 18.5, the BMI value is set as 0;

when the subject's BMI is 18.5 or more but less than 24, the BMI value is set as 1, and c is any value selected from -0.306744 to 0.3390827;

when the subject's BMI is 24 or more but less than 28, the BMI value is set as 1, and c is any value selected from 0.1934385 to 0.8774874; and

when the subject's BMI is 28 or more, the BMI value is set as 1, and c is any value selected from 0.5968209 to 1.3646449.

11. The system according to item 2, wherein
the grouping basis pre-stored in the grouping module is:

when the calculated probability (p) of a subject suffering from polycystic ovary syndrome is less than 10%, the risk of a subject suffering from polycystic ovary syndrome is low;

when the calculated probability (p) of a subject suffering from polycystic ovary syndrome is 10% or more but less than 50%, the risk of a subject suffering from polycystic ovary syndrome is moderate; and

when the calculated probability (p) of a subject suffering from polycystic ovary syndrome is 50% or more, the risk of a subject suffering from polycystic ovary syndrome is high.

12. A method for screening polycystic ovary syndrome, comprising:

a data collection step for obtaining anti-Mullerian hormone (AMH) level in a subject, collecting upper limit of the number of menstrual cycle days voluntarily provided by the subject, and collecting BMI of the subject; and

a step for calculating a probability of suffering from polycystic ovary syndrome, which is used to calculate the above data information obtained from a data collection module, so as to calculate a probability (p) of a subject suffering from polycystic ovary syndrome.

13. The method according to item 12, further comprising:
a grouping step for pre-storing default grouping parameters for polycystic ovary syndrome, grouping the calculated probability (p) of a subject suffering from polycystic ovary syndrome based on the grouping parameters, thereby grouping the risk of a subject suffering from polycystic ovary syndrome.

14. The method according to item 12 or 13, wherein
in the step for calculating a probability of suffering from polycystic ovary syndrome, the probability (p) of a subject suffering from polycystic ovary syndrome is calculated by converting data of the anti-Mullerian hormone (AMH) level, upper limit of the number of menstrual cycle days, and BMI of the subject into polytomous variables.

15. The method according to item 12 or 13, wherein
the anti-Mullerian hormone (AMH) level refers to a concentration of AMH in venous blood of a female subject on any day of the menstrual cycle.

16. The method according to item 12 or 13, wherein

in the step for calculating a probability of suffering from polycystic ovary syndrome, the levels of anti-Mullerian hormone (AMH) are converted into five-categorical variables, that is to say,

the AMH levels are divided into the following five groups: the subjects' AMH levels are less than 2.5 ng/ml, the subjects' AMH levels are 2.5 ng/ml or more but less than 5 ng/ml, the subjects' AMH levels are 5 ng/ml or more but less than 7.5 ng/ml, the subjects' AMH levels are 7.5 ng/ml or more but less than 10 ng/ml, and the subjects' AMH levels are 10 ng/ml or more.

17. The method according to item 12 or 13, wherein

in the step for calculating a probability of suffering from polycystic ovary syndrome, the upper limits of the number of menstrual cycle days of the subjects are converted into five-categorical variables, that is to say,
the upper limits of the number of menstrual cycle days of the subjects are divided into the following five groups: the upper limits of the number of menstrual cycle days of the subjects are less than 35 days, the upper limits of the number of menstrual cycle days of the subjects are 35 days or more but less than 45 days, the upper limits of the number of menstrual cycle days of the subjects are 45 days or more but less than 60 days, the upper limits of the number of menstrual cycle days of the subjects are 60 days or more but less than 90 days, and the upper limits of the number of menstrual cycle days of the subjects are 90 days or more.

18. The method according to item 12 or 13, wherein

in the step for calculating a probability of suffering from polycystic ovary syndrome, the BMIs of the subjects are converted into four-categorical variables, that is to say,
the BMIs of the subjects are divided into the following four groups: BMIs of the subjects are less than 18.5, BMIs of the subjects are 18.5 or more but less than 24, BMIs of the subjects are 24 or more but less than 28, and BMIs of the subjects are 28 or more.

19. The method according to item 12 or 13, wherein
in the step for calculating a probability of suffering from polycystic ovary syndrome, a formula for calculating a probability (p) of suffering from polycystic ovary syndrome is pre-stored, and the formula is obtained by fitting the polytomous variables converted from the data of the anti-Mullerian hormone (AMH) level, upper limit of the number of menstrual cycle days, and BMI of the subject in the existing database.
20. The method according to item 19, wherein

the above formula is the following Formula I:

$$p=1/[1+e^{-(i+a*\text{AMH}+b*\text{upper limit of the number of menstrual cycle days}+c*\text{BMI})}] \quad (\text{Formula I})$$

wherein p is the calculated probability of a subject suffering from polycystic ovary syndrome, and a, b, c and i are unitless parameters;
in the step for calculating a probability of suffering from polycystic ovary syndrome, the values of a, b and c are obtained based on the anti-Mullerian hormone (AMH) level, upper limit of the number of menstrual cycle days, and BMI of the subject and are plugged into Formula I for calculation,
in the calculation, the value of AMH, the upper limit of the number of menstrual cycle days, or BMI is set as 0 or 1.

21. The method according to item 20, wherein

i is any value selected from -4.255406 to -3.56205;
when the subject's AMH level is less than 2.5 ng/ml, the AMH value is set as 0;
when the subject's AMH level is 2.5 ng/ml or more but less than 5 ng/ml, the AMH value is set as 1, and a is any value selected from 0.3982787 to 0.846642;
when the subject's AMH level is 5 ng/ml or more but less than 7.5 ng/ml, the AMH value is set as 1, and a is any value selected from 1.3775495 to 1.8533111;
when the subject's AMH level is 7.5 ng/ml or more but less than 10 ng/ml, the AMH value is set as 1, and a is any value selected from 2.0095086 to 2.5992375;
when the subject's AMH level is 10 ng/ml or more, the AMH value is set as 1, and a is any value selected from 2.4552789 to 3.0324161;
when the upper limit of the number of menstrual cycle days of the subject is less than 35 days, the value of the upper limit of the number of menstrual cycle days is set as 0;
when the upper limit of the number of menstrual cycle days of the subject is 35 days or more but less than 45 days, the value of the upper limit of the number of menstrual cycle days is set as 1, and b is any value selected from 1.1836336 to 1.6602398;
when the upper limit of the number of menstrual cycle days of the subject is 45 days or more but less than 60 days, the value of the upper limit of the menstrual cycle days is set as 1, and b is any value selected from 1.6734536 to 2.1734828;

when the upper limit of the number of menstrual cycle days of the subject is 60 days or more but less than 90 days, the value of the upper limit of the number of menstrual cycle days is set as 1, and b is any value selected from 1.7119305 to 2.4218832;

when the upper limit of the number of menstrual cycle days of the subject is 90 days or more, the value of the upper limit of the number of menstrual cycle days is set as 1, and b is any value selected from 2.0049859 to 2.682179;

when the subject's BMI is less than 18.5, the BMI value is set as 0;

when the subject's BMI is 18.5 or more but less than 24, the BMI value is set as 1, and c is any value selected from -0.306744 to 0.3390827;

when the subject's BMI is 24 or more but less than 28, the BMI value is set as 1, and c is any value selected from 0.1934385 to 0.8774874; and

when the subject's BMI is 28 or more, the BMI value is set as 1, and c is any value selected from 0.5968209 to 1.3646449.

22. The method according to item 13, wherein
the grouping basis pre-stored in the grouping step is:

when the calculated probability (p) of a subject suffering from polycystic ovary syndrome is less than 10%, the risk of a subject suffering from polycystic ovary syndrome is low;

when the calculated probability (p) of a subject suffering from polycystic ovary syndrome is less than 50%, the risk of a subject suffering from polycystic ovary syndrome is moderate; and

when the calculated probability (p) of a subject suffering from polycystic ovary syndrome is 50% or more, the risk of a subject suffering from polycystic ovary syndrome is high.

**Effects of the Invention**

Although there is already a method and system for screening polycystic ovary syndrome with four indicators developed by the applicant, a simpler and more accurate model is still needed to develop in this field. Therefore, this application has established a mathematical model with three parameters, i.e., a model relating to AMH, the number of menstrual cycle days, and BMI. This model avoids the need for immunoassay-based androstenedione levels to screen for polycystic ovary syndrome in the prior art.

Compared with the prior art, the present application has the following beneficial effects: firstly, the system constructed in the present application only uses three parameters: AMH, the number of menstrual cycle days, and BMI; avoiding the issue of inaccuracy in Immunoassay of Androgen, thereby further reducing testing time and cost, and improving testing efficiency and accuracy. Secondly, the applicant of this application used real-world research with a large number of samples and external validation, indicating that the optimized three-indicator PCOS model has the same predictive effect as the previous four-indicator model. It can be seen that, the screening model for polycystic ovary syndrome established by the applicant helps to screen and diagnose polycystic ovary syndrome more quickly and conveniently in clinical practice, and may further elucidate the etiology of polycystic ovary syndrome; that is, AMH is located upstream of the androgen signaling pathway, which is a more critical indicator for screening PCOS than androgen level. Using the system of this application, the probability (p) of suffering from polycystic ovary syndrome for a subject can be calculated, and based on the default grouping parameters of polycystic ovary syndrome stored in the system, the probability (p) of a subject suffering from polycystic ovary syndrome can be grouped to determine the risk of a subject suffering from polycystic ovary syndrome.

SPECIFIC EMBODIMENTS

In this application, menstrual cycle refers to the number of days each menstrual cycle lasts, typically ranging from 3 to 7 days. Menstrual cycle refers to the time interval between the first day of two menstrual cycles, and the upper limit of the number of menstrual cycle days for a subject is voluntarily provided by the subject. For example, if the subject's menstrual cycle is usually 30-90 days based on past experience, the upper limit of the menstrual cycle days obtained in this application is 90 days.

Anti-Mullerian hormone (AMH) is a hormone secreted by the granulosa layer cells of small ovarian follicles. During fetal life, a female baby begins to produce AMH at 36 weeks. The more small follicles in the ovaries, the higher the concentration of AMH will be. On the contrary, as follicles gradually deplete with age and various factors, the concentration of AMH will also decrease, and as they approach menopause, AMH gradually tends to zero.

BMI (Body Mass Index), also known as body constitutional index, is a number obtained by dividing the number of kilograms of body weight by the square of the number of meters of height. It is a commonly used international standard to measure the degree of obesity and health of the human body, and is mainly used for statistical purposes, when we need to compare and analyze the health effects of a person's weight on people of different heights, BMI value is a neutral and reliable indicator.

Continuous variables: in statistics, variables can be divided into continuous variables and categorical variables based on whether their values are continuous. A variable that can take any value within a certain interval is called a continuous variable, and its value is continuous, and a range between two adjacent values can be infinitely divided, resulting in an infinite number of values. For example, the specifications and dimensions of production parts, as well as the height, weight, and chest circumference etc. obtained from somatometry are continuous variables, and their values can only be obtained through measurement or metrological methods. On the contrary, if its numerical value can only be calculated in natural or integer units, it is a discrete variable. For example, the number of enterprises, number of employees, number of equipment, etc. can only be counted in units of measurement, and the values of these variables are generally obtained by counting methods.

Categorical variables refer to variables such as geographical location and demographics, which serve to group survey respondents. Descriptive variables describe the differences between a certain customer group and other customer groups. Most categorical variables are descriptive variables. Categorical variables can be divided into two categories: unordered categorical variables and ordered categorical variables. Particularly, unordered categorical variables refer to the lack of degree and order differences between the classified categories or attributes, and can be further divided into two categories: ① binomial classifications, such as gender (male, female), drug reactions (negative and positive), etc.; ② multiple classifications, such as blood type (O, A, B, and AB), occupation (industrial, agricultural, commercial, academic, and military), etc. There is a degree of difference between the categories of ordered categorical variables. For example, the test results of urine glucose are classified according to -, $\pm$, +, ++, and +++; the therapeutic effect is classified according to cure, significant effect, improvement, and ineffective. For ordered categorical variables, they should be grouped in hierarchical order. That is to say, the number of observation units in each group should be counted, a frequency table for ordered variables (each level) should be compiled, and the obtained data is called hierarchical data.

The type of variable is not fixed, and according to the research purpose, various variables can be transformed between each other. For example, hemoglobin volume (g/L) is originally a numerical variable, if it is divided into two categories based on normal and low hemoglobin, the data can be analyzed according to binomial classifications; and if it is divided into five levels: severe anemia, moderate anemia, mild anemia, normal and increased hemoglobin, the data can be analyzed based on the level. Sometimes, categorical data can also be quantified. For example, the patient's nausea response can be represented by 0, 1, 2, or 3, and it can be analyzed based on numerical variable data (quantitative data).

Logistic regression is a generalized linear regression analysis model commonly used in fields such as data mining, automatic disease diagnosis, and economic prediction; for example, exploring the risk factors that cause diseases, and predicting the probability of disease occurrence based on these risk factors. Taking the analysis of gastric cancer as an example, two groups of people were selected, i.e., one being the gastric cancer group, and the other being the non-gastric cancer group. The two groups of people must have different physical signs and lifestyles. Therefore, the dependent variable is whether there is gastric cancer, with a value of "yes" or "no", and the independent variables can comprise many factors, such as age, gender, dietary habits, *Helicobacter pylori* infection, etc. The independent variables can be either continuous or categorical. Then, the weights of independent variables can be obtained through logistic regression analysis, thereby roughly understanding which factors are risk factors for gastric cancer. At the same time, the probability of suffering from cancer for a subject can be predicted based on risk factors and this weight value. The dependent variable of logistic regression may belong to binomial or multiple classifications.

The data fitting model used herein is a logistic regression model, and the absolute sizes of the coefficients in the regression model are penalized according to $\lambda$ values. The greater the penalty, the closer the estimation of weaker factors approaches zero, thus only the strongest predictive variable remains in the model.

The least absolute shrinkage and selection operator regression (commonly referred to as Lasso regression) is a compression estimation method based on the idea of reducing the variable set (order reduction). It can compress the coefficients of variables and make certain regression coefficients 0 by constructing a penalty function, thereby achieving the goal of variable selection. It is an algorithm that utilizes penalty functions to improve prediction ability of a model. The algorithm uses 1-norm constraints to not only solve high-dimensional and collinearity problems, but also make the established model "sparse", which means the algorithm has the effect of automatically selecting wavelengths in modeling.

10 fold cross validation, also known as 10-fold cross validation, is a commonly used testing method to test the accuracy of an algorithm. During validation, the dataset is divided into ten parts, with 9 of them being used as training data and 1 as testing data in turn for experimentation. Each experiment will yield the corresponding accuracy (or error rate). The average of the accuracy (or error rate) of 10 results is used as an estimate of algorithm accuracy. Generally, multiple 10-fold cross validations (such as 10 times of 10-fold cross validation) are required, and then the average is calculated as an estimate of algorithm accuracy. The reason why 10-fold cross validation chooses to divide the dataset into 10 parts, because after extensive experiments by using a large number of datasets and different learning techniques, indicating that 10-fold is an appropriate choice for obtaining the best error estimate, and there are also some theoretical grounds to prove this.

Collinearity is also known as synteny. In statistics, collinearity refers to multicollinearity. Multicollinearity refers to the distortion or difficulty in accurately estimating of a model due to the existence of precise or highly correlated relationships between the explanatory variables in a linear regression model. Generally speaking, due to limitations in economic data, improper model design leads to a general correlation between explanatory variables in the designed matrix. The situation of complete collinearity is not common, and generally a certain extent of collinearity (i.e., approximate collinearity) will occur.

Overfitting is "an analysis result too closely or accurately corresponding to a specific dataset, and thus it may not be able to fit other data or reliably predict future observations". An overfitting model is a statistical model that contains multiple parameters which may exceed the reasonable range of data. The essence of overfitting is to unconsciously extract some residual changes (i.e., noise), as if the changes represent the basic model structure. In other words, the model remembers a large number of examples rather than learning attention features. The possibility of overfitting not only depends on the number of parameters and data, but also on the consistency of the model structure and data shape, as well as the size of the model error as compared to the expected level of noise or data error. Even if the fitted model does not have too many parameters, it can be expected that the performance of the fitted relationship on the new dataset will be worse than on the fitted dataset (sometimes this situation occurs and is referred to as shrinkage).

Receiver operating characteristic curve (ROC curve) is also known as sensitivity curve. The reason for this name is that each point on the curve reflects the same receptivity, and they are all responses to the same signal stimulus, just results obtained under several different judgment criteria. The receiver operating characteristic curve is a coordinate graph composed of false alarm probability as the horizontal axis and hit probability as the vertical axis, and the curve drawn by different results obtained from different judgment criteria under specific stimulus conditions.

This application provides a system for screening polycystic ovary syndrome, which comprises:

a data collection module for obtaining anti-Mullerian hormone (AMH) level in a subject, collecting upper limit of the number of menstrual cycle days voluntarily provided by the subject, and collecting BMI of the subject; and
a module for calculating a probability of suffering from polycystic ovary syndrome, which is used to calculate the above data information obtained from the data collection module, so as to calculate a probability (p) of a subject suffering from polycystic ovary syndrome. In the module for calculating a probability of suffering from polycystic ovary syndrome, the probability (p) of a subject suffering from polycystic ovary syndrome is calculated by converting data of the anti-Mullerian hormone (AMH) level, upper limit of the number of menstrual cycle days, and BMI of the subject into polytomous variables. Particularly, the anti-Mullerian hormone (AMH) level refers to a concentration of AMH in venous blood of a female subject on any day of the menstrual cycle. For example, if the menstrual cycle of the subject is 28 days, the AMH level can be the concentration of AMH in venous blood on the first day of the menstrual cycle, the concentration of AMH in venous blood on day 10 of the menstrual cycle, or the concentration of AMH in venous blood on day 28 of the monthly menstrual cycle.

In the module for calculating a probability of suffering from polycystic ovary syndrome, the inventors of this application conducted in-depth research, and converted the levels of anti-Mullerian hormone (AMH) into five-categorical variables by exploring the distribution of independent variables and outcome variables. That is to say, the AMH levels are divided into the following five groups: the subjects' AMH levels are less than 2.5 ng/ml, the subjects' AMH levels are 2.5 ng/ml or more but less than 5 ng/ml, the subjects' AMH levels are 5 ng/ml or more but less than 7.5 ng/ml, the subjects' AMH levels are 7.5 ng/ml or more but less than 10 ng/ml, and the subjects' AMH levels are 10 ng/ml or more.

In the module for calculating a probability of suffering from polycystic ovary syndrome, the inventors of this application conducted in-depth research, and converted the upper limits of the number of menstrual cycle days of the subjects into five-categorical variables by exploring the distribution of independent variables and outcome variables. That is to say, the upper limits of the number of menstrual cycle days of the subjects are divided into the following five groups: the upper limits of the number of menstrual cycle days of the subjects are less than 35 days, the upper limits of the number of menstrual cycle days of the subjects are 35 days or more but less than 45 days, the upper limits of the number of menstrual cycle days of the subjects are 45 days or more but less than 60 days, the upper limits of the number of menstrual cycle days of the subjects are 60 days or more but less than 90 days, and the upper limits of the number of menstrual cycle days of the subjects are 90 days or more.

In the module for calculating a probability of suffering from polycystic ovary syndrome, the inventors of this application conducted in-depth research, and converted the subject's BMIs into four-categorical variables by exploring the distribution of independent variables and outcome variables. That is to say, the BMIs of the subjects are divided into the following four groups: BMIs of the subjects are less than 18.5, BMIs of the subjects are 18.5 or more but less than 24, BMIs of the subjects are 24 or more but less than 28, and BMIs of the subjects are 28 or more.

After transforming the above three variables into different polytomous variables, the non-linear relationship between the independent variable and the outcome variable can be converted into a linear relationship by using such polytomous variables for data analysis, the probability of a subject suffering from polycystic ovary syndrome can be calculated more accurately, and the model has a better stability.

In the module for calculating a probability of suffering from polycystic ovary syndrome, a formula for calculating a probability (p) of suffering from polycystic ovary syndrome is pre-stored, and the formula is obtained by fitting the polytomous variables converted from the data of the anti-Mullerian hormone (AMH) level, upper limit of the number of menstrual cycle days, and BMI of the subject in the existing database. And then the probability (p) of a subject suffering from polycystic ovary syndrome is grouped according to the grouping criteria.

In this application, the existing database refers to a database of subjects who are currently receiving treatment or have previously received treatment and meet the inclusion and exclusion criteria mentioned below, which can be obtained. There is no agreement on the number of the samples in the database, and of course, the larger the number of samples in the database, the better. For example, it may be 100 subjects, 200 subjects, 300 subjects, preferably 400 subjects or more, more preferably 500 subjects or more. In a particular example, an existing database consisting of 11720 samples is used.

Analysis data: for a total of 21219 ovulation induction treatment cycles visited the Reproductive Medicine Center of Peking University Third Hospital from January to December in 2019, after excluding cycles with incomplete records such as menstrual cycle, BMI, testosterone, androstenedione, and antral follicle counting (AFC), 11720 treatment cycles were included in the final analysis. The menstrual cycle days in this study refer to the upper limit of the duration of the menstrual cycle. For example, if the patient's menstrual cycle is 30-90 days, 90 days will be used for analysis. For unidentified data in our analysis, there is no need for informed consent from patients, which is in line with the Helsinki Declaration.

In the module for calculating a probability of suffering from polycystic ovary syndrome, the following Formula (I) is used to calculate the probability (p) of a subject suffering from polycystic ovary syndrome:

$$p=1/[1+e^{-(i+a*AMH+b*\text{upper limit of the number of menstrual cycle days}+c*BMI)}] \quad (\text{Formula I})$$

wherein p is the calculated probability of a subject suffering from polycystic ovary syndrome, and a, b, c and i are unitless parameters;

in the module for calculating a probability of suffering from polycystic ovary syndrome, the values of a, b and c are obtained based on the anti-Mullerian hormone (AMH) level, upper limit of the number of menstrual cycle days, and BMI of the subject and are plugged into Formula I for calculation,

in the calculation, the value of AMH, the upper limit of the number of menstrual cycle days, or BMI is set as 0 or 1.

Furthermore, i is any value selected from -4.255406 to -3.56205; when the subject's AMH level is less than 2.5 ng/ml, the AMH value is set as 0; when the subject's AMH level is 2.5 ng/ml or more but less than 5 ng/ml, the AMH value is set as 1, and a is any value selected from 0.3982787 to 0.846642; when the subject's AMH level is 5 ng/ml or more but less than 7.5 ng/ml, the AMH value is set as 1, and a is any value selected from 1.3775495 to 1.8533111; when the subject's AMH level is 7.5 ng/ml or more but less than 10 ng/ml, the AMH value is set as 1, and a is any value selected from 2.0095086 to 2.5992375; when the subject's AMH level is 10 ng/ml or more, the AMH value is set as 1, and a is any value selected from 2.4552789 to 3.0324161; when the upper limit of the number of menstrual cycle days of the subject is less than 35 days, the value of the upper limit of the number of menstrual cycle days is set as 0; when the upper limit of the number of menstrual cycle days of the subject is 35 days or more but less than 45 days, the value of the upper limit of the number of menstrual cycle days is set as 1, and b is any value selected from 1.1836336 to 1.6602398; when the upper limit of the number of menstrual cycle days of the subject is 45 days or more but less than 60 days, the value of the upper limit of the menstrual cycle days is set as 1, and b is any value selected from 1.6734536 to 2.1734828; when the upper limit of the number of menstrual cycle days of the subject is 60 days or more but less than 90 days, the value of the upper limit of the number of menstrual cycle days is set as 1, and b is any value selected from 1.7119305 to 2.4218832; when the upper limit of the number of menstrual cycle days of the subject is 90 days or more, the value of the upper limit of the number of menstrual cycle days is set as 1, and b is any value selected from 2.0049859 to 2.682179; when the subject's BMI is less than 18.5, the BMI value is set as 0; when the subject's BMI is 18.5 or more but less than 24, the BMI value is set as 1, and c is any value selected from -0.306744 to 0.3390827; when the subject's BMI is 24 or more but less than 28, the BMI value is set as 1, and c is any value selected from 0.1934385 to 0.8774874; and when the subject's BMI is 28 or more, the BMI value is set as 1, and c is any value selected from 0.5968209 to 1.3646449.

The default grouping parameters for polycystic ovary syndrome are pre-stored in the grouping module of this application. The grouping basis pre-stored in the grouping module is: when the calculated probability (p) of a subject suffering from polycystic ovary syndrome is less than 10%, the risk of a subject suffering from polycystic ovary syndrome is low; when the calculated probability (p) of a subject suffering from polycystic ovary syndrome is 10% or more but less than 50%, the risk of a subject suffering from polycystic ovary syndrome is moderate; and when the calculated probability (p) of a subject suffering from polycystic ovary syndrome is 50% or more, the risk of a subject suffering from polycystic ovary syndrome is high.

In another particular embodiment of the present application, the present application also relates to a method for screening polycystic ovary syndrome, which comprises: a data collection step for obtaining anti-Mullerian hormone (AMH) level in a subject, collecting upper limit of the number of menstrual cycle days voluntarily provided by the subject, and collecting BMI of the subject; and a step for calculating a probability of suffering from polycystic ovary syndrome, which is used to calculate the above data information obtained from a data collection module, so as to calculate a probability (p) of a subject suffering from polycystic ovary syndrome.

As mentioned above, the specific contents of the steps carried out in the method of this application can refer to the steps of the various modules of the system involved in this application for the acquisition, grouping and treatment method of the anti-Mullerian hormone (AMH) level in a subject, the upper limit of the number of menstrual cycle days voluntarily provided by the subject, and the subjects' BMI.

EXAMPLES

**Selection of Experimental Data**

In this example, case data from Peking University Third Hospital was used. The applicant of this application collected records of 21219 subjects who underwent ovulation induction cycles from January to December in 2019, and excluded the cycle data of the following subjects after screening: 3289 subjects without menstrual cycle data, 150 subjects without body mass index (BMI) information, and 3180 subjects without testosterone levels, 31 subjects without androstenedione levels, and 3849 subjects without antral follicle count (AFC) information. Finally, cycle data of 11720 subjects were selected for statistical analysis, and used to construct the system of this application in this example.

In this example, the upper limit of the number of menstrual cycle days refers to the upper limit of the duration of the menstrual cycle. For example, if the subject provides the past menstrual cycles of 30-90 days during the treatment period, 90 days will be used as the upper limit for the number of menstrual cycle days.

The analysis conducted for this example does not involve patient identity information, and does not require patient informed consent, which is in line with the Helsinki Declaration.

**Clinical Screening of PCOS**

According to the 2003 Rotterdam criteria, Group, R.E.A.S.P.c.w. Revised 2003 concerns on diagnostic criteria and long-term health risks related to polycystic ovary syndrome. Fertil Steril 81, 19-25 (2004), the diagnosis of PCOS based on this standard requires at least two of the following symptoms in a subject: (1) ovulation dysfunction (i.e., sparse ovulation and/or anovulation); (2) clinical manifestations of hyperandrogenism (high levels of testosterone or androstenedione in blood tests) or excessive androgen; (3) polycystic ovary confirmed by ultrasound examination. After excluding phenotypically similar androgenic disorders such as congenital adrenal hyperplasia, androgen secreting tumors, Cushing's syndrome, thyroid dysfunction, and hyperprolactinemia, the final diagnosis of PCOS is made.

The clinical manifestations of hyperandrogenism refer to acne, androgenic alopecia, or hirsutism. Hyperandrogenism refers to an increase in serum total testosterone or androstenone levels. In this example, the diagnosis of hirsutism is based on the following criteria: a modified Ferriman Galwey score >4, or hair growth involving the upper lip, thighs, and lower abdomen, with a hair growth score >2. In rare cases where androgen secreting tumors are suspected (such as when subjects experience obvious viral infections or rapid onset of PCOS related symptoms), it is useful to measure the androgen levels in the subjects.

Particularly, menstrual cycles that last more than 35 days but less than 6 months are diagnosed as oligomenorrhea. Amenorrhea refers to the absence of menstruation for more than 6 months after the formation of a periodic pattern. Polycystic ovaries in ultrasound examination are defined as at least one ovary containing 12 or more follicles with a diameter of 2-9 mm or an ovarian volume greater than 10 mL. A single ovary can be diagnosed as polycystic ovary if it meets one or both of the above two definitions. Hyperprolactinemia is diagnosed when the serum prolactin (PRL) level exceeds 25 ng/mL twice.

**Antral Follicle Counting and Endocrine Measurement**

In this example, in the menstrual cycle or on day 2 of the menstrual cycle, the number of antral follicles with a diameter of 2-10 mm in the two ovaries of a subject was counted through transvaginal ultrasound scanning. On the same day, venous blood from the subject was collected to measure the concentrations of prolactin (PRL), luteinizing hormone (LH), testosterone, androstenedione, and serum estradiol (E2). Blood samples were collected every day for measuring AMH during the menstrual cycle. Blood samples were collected to immediately invert them five times, then centrifuging for further endocrine evaluation.

Serum levels of PRL, LH, testosterone, androstenedione, and $E_2$ were tested by using the Siemens Immunoassay 2000 system (Siemens Healthcare Diagnostics, Shanghai, China). The quality control of PRL, LH, testosterone, androstenedione, and $E_2$ was provided by the Bio RAD laboratory (Hercules, California, USA; Lyphochek Immunoassay Plus Control, Level III, Catalog No. 370, Batch No. 40370). The quality control provided with the kit and an ultra-sensitive two-point ELISA (Ansh Labs LLC; Webster, TX, USA) were used to measure serum AMH concentration. The coefficient of variation for Level 3 quality control, AMH, PRL, and LH measurements is less than 6%, while the coefficient of variation for $E_2$, androstenedione, and testosterone is less than 10%.

## Data Analysis

All analyses in this example were conducted by using SAS JMP Pro (version 14.2; SAS Institute, Cary, NC, USA), and in the analysis if $p<0.05$, it is considered statistically significant. Normally distributed variables were displayed as mean and standard deviation, while non-normally distributed variables were displayed as median and quartile. For variable selection, seven or eight variables were put into the selection process. Least Absolute Shrinkage and Selection Operator Regression (LASSO) were used to minimize the potential collinearity of variables and overfitting of variables measured from the same subject.

In this example, the inventors used L1-penalized Least Absolute Shrinkage and Selection Operator Regression for multivariate analysis, and conducted internal validation by using 10 fold cross validation. This is a logistic regression model, which penalizes the absolute size of the coefficients in the regression model based on $\lambda$ value. The greater the penalty, the closer the estimation of weaker factors approaches zero, thus only the strongest predictive variable remains in the model. The most predictive covariate is selected by the minimum value ($\lambda$min). Subsequently, the variables determined through LASSO regression analysis will be input into the logistic regression model, and the consistently and statistically significant variables will be used to construct the PCOS screening model.

In this example, the inventors evaluated the performance of the PCOS model by using receiver-operator characteristic curve (AUC) and area under sensitivity and specificity.

## Variable Selection

Firstly, Table 1 lists the basic characteristics of the variables collected in the example. These indicators are of great significance in univariate analysis when screening PCOS. To analyze the correlation between variables, continuous variables are converted into categorical variables. The grouping criteria for independent variables are mainly based on data exploration before analysis, and combined with the clinical experience of the inventors of this application. In three different models, the grouping criteria for each independent variable remain unchanged.

Table 1: Variable Characteristics

| Variables | PCOS (n=1225) | Non-PCOS (n=10495) | p |
|---|---|---|---|
| Age (years) | 31.4±5.0 | 33.6±5.2 | <0.0001 |
| BMI (kg/m$^2$) | 24.1 (21.5-26.8) | 22.2 (20.3-24.5) | <0.0001 |
| AMH (ng/mL) | 6.4 (3.5-10.1) | 2.1 (1.0-3.8) | <0.0001 |
| upper limit of the number of menstrual cycle days (days) | 40 (30-62.5) | 29 (28-30) | <0.0001 |
| TES (nmol/L) | 0.7 (0.7-1.1) | 0.7 (0.7-0.7) | <0.0001 |
| AND (nmol/L) | 8.9±4.9 | 5.8±3.1 | <0.0001 |
| AFC | 10 (6-14) | 10 (7-15) | <0.0001 |

Particularly, BMI in Table 1 represents body mass index; AMH represents the level of anti-Mullerian hormone; TES represents the level of testosterone; AND represents the level of androstenedione; AFC represents the number of antral follicles.

In the construction of the entire model, the upper limit of the number of menstrual cycle days, AMH level, and BMI of the subjects were used as variables.

To this end, the inventors first divided the data of all 11720 subjects into an internal validation group and an external validation data group in a ratio of 70% to 30%. In the internal validation group, the optimal model (i.e. PCOS-3 model) is determined by combining LASSO regression with 10 fold logistic regression. The raw data and corresponding predicted data were used to calculate the probability of subjects suffering from PCOS.

Table 2 shows the contribution of each variable in the PCOS-3 model. Particularly, the main effect refers to the contribution of a single indicator to the model, while the total effect is the sum of the contribution of a single indicator itself + the interaction between a single indicator and other indicators to the model. The results in Table 2 show that, the main effects of each predictor on the PCOS-3 model are as follows: AMH, 47.0%; upper limit of the number of menstrual cycle days, 34.5%; and BMI, 4.3%. Compared with the model (PCOS-4) constructed in the previous patent CN113035354B of this applicant, the contribution of AMH in the PCOS-3 model of this application has increased in both the main and total effects. The main effect of AMH in PCOS-3 exceeds 3.7% of A4 in the PCOS-4 model.

Table 2: Contribution of each predictive variable in the PCOS-3 and PCOS-4 models constructed by the example

|  | Main effect (%) | | Total effect (%) | |
|---|---|---|---|---|
|  | PCOS-3 | PCOS-4 | PCOS-3 | PCOS-4 |
| AMH | 47 | 41.2 | 58.9 | 52.3 |
| UMCL | 34.5 | 35.2 | 46.2 | 45.8 |
| BMI | 4.3 | 4.3 | 7.9 | 8.6 |
| A4 | -- | 3.7 | -- | 7.2 |

Table 3 shows the AUC, sensitivity, and specificity of modeling data, internal validation data, and external validation data in the PCOS-3 model and the PCOS-4 model. The AUC of modeling data, internal validation data, and external validation data in the PCOS-3 model is 0.850, 0.851, and 0.841, respectively. It can be seen that, there is no significant difference in AUC, sensitivity, and specificity between the two models, as their 95% confidence intervals overlap, indicating similar performance between the two models.

Table 3: Performance comparison between PCOS-3 Model and PCOS-4 Model

|  | Modeling data | | Internal validation data | | External validation data | |
|---|---|---|---|---|---|---|
|  | PCOS-3 | PCOS-4 | PCOS-3 | PCOS-4 | PCOS-3 | PCOS-4 |
| AUC (95% CI) | 0.850 (0.842, 0.858) | 0.855 (0.838-0 .870) | 0.851 (0.828, 0.874) | 0.848 (0.791-0.8 91) | 0.841 (0.826, 0.856) | 0.846 (0.812-0.8 75) |
| Sensitivity (95% CI) | 0.339 (0.308, 0.370) | 0.362 (0.331-0 .395) | 0.384 (0.294, 0.482) | 0.394 (0.303-0.4 92) | 0.383 (0.324, 0.445) | 0.383 (0.324-0.4 45) |
| Specificity (95% CI) | 0.981 (0.978, 0.984) | 0.981 (0.997-0 .983) | 0.983 (0.972, 0.990) | 0.985 (0.974-0.9 91) | 0.981 (0.974, 0.986) | 0.981 (0.974-0.9 86) |

In summary, based on the confirmed PCOS-3 model in this example, a formula for calculating a probability (p) of suffering from polycystic ovary syndrome can be obtained, i.e., the following Formula I. The probability (p) of a subject suffering from PCOS can be calculated by this Formula I based on the subject's anti-Mullerian hormone (AMH) level, upper limit of the number of menstrual cycle days, and BMI.

$$\text{Formula I: } p = 1/[1 + e^{-(i + a*AMH + b*\text{upper limit of the number of menstrual cycle days} + c*BMI)}]$$

wherein p is the calculated probability (p) of a subject suffering from PCOS, and a, b, c and i are unitless parameters; in the module for calculating a probability of suffering from polycystic ovary syndrome, the values of a, b and c are obtained based on the anti-Mullerian hormone (AMH) level, upper limit of the number of menstrual cycle days, and BMI of the subject and are plugged into Formula I for calculation,

in the calculation, the value of AMH, the upper limit of the number of menstrual cycle days, or BMI is set as 0 or 1. i is any value selected from -4.255406 to -3.56205; when the subject's AMH level is less than 2.5 ng/ml, the AMH value is set as 0; when the subject's AMH level is 2.5 ng/ml or more but less than 5 ng/ml, the AMH value is set as 1, and a is any value selected from 0.3982787 to 0.846642; when the subject's AMH level is 5 ng/ml or more but less than 7.5 ng/ml, the AMH value is set as 1, and a is any value selected from 1.3775495 to 1.8533111; when the subject's AMH level is 7.5 ng/ml or more but less than 10 ng/ml, the AMH value is set as 1, and a is any value selected from 2.0095086 to 2.5992375; when the subject's AMH level is 10 ng/ml or more, the AMH value is set as 1, and a is any value selected from 2.4552789 to 3.0324161; when the upper limit of the number of menstrual cycle days of the subject is less than 35 days, the value of the upper limit of the number of menstrual cycle days is set as 0; when the upper limit of the number of menstrual cycle days of the subject is 35 days or more but less than 45 days, the value of the upper limit of the number of menstrual cycle days is set as 1, and b is any value selected from 1.1836336 to 1.6602398; when the upper limit of the number of menstrual cycle days of the subject is 45 days or more but less than 60 days, the value of the upper limit of the menstrual cycle days is set as 1, and b is any value selected from 1.6734536 to 2.1734828; when the upper limit of the number of menstrual cycle days of the subject is 60 days or more but less than 90 days, the value of the upper limit of the menstrual cycle days is set as 1, and b is any value selected from 1.7119305 to 2.4218832; when the upper limit of the number of menstrual cycle days of the subject is 90 days or more, the value of the upper limit of the number of menstrual cycle days is set as 1, and b is any value selected from 2.0049859 to 2.682179; when the subject's BMI is less than 18.5, the BMI value is set as 0; when the subject's BMI is 18.5 or more but less than 24, the BMI value is set as 1, and c is any value selected from -0.306744 to 0.3390827; when the subject's BMI is 24 or more but less than 28, the BMI value is set as 1, and c is any value selected from 0.1934385 to 0.8774874; and when the subject's BMI is 28 or more, the BMI value is set as 1, and c is any value selected from 0.5968209 to 1.3646449.

The relationship between the predicted probability calculated by using the above Formula I and the actual occurrence rate of PCOS is that: the actual occurrence rate of PCOS increases with the increase of predicted probability. When the calculated probability (p) of a subject suffering from polycystic ovary syndrome is less than 10%, the risk of a subject suffering from polycystic ovary syndrome is low; when the calculated probability (p) of a subject suffering from polycystic ovary syndrome is 10% or more but less than 50%, the risk of a subject suffering from polycystic ovary syndrome is moderate; and when the calculated probability (p) of a subject suffering from polycystic ovary syndrome is 50% or more, the risk of a subject suffering from polycystic ovary syndrome is high.

Although the incidence rate of PCOS is high, the diagnosis of PCOS in the world is complex. The Rotterdam standard is the most commonly used in the world. It needs a comprehensive evaluation by combining physical examination, medical history, ultrasound examination and blood test, and has high requirements on the diagnostic level of doctors, thereby being difficult to promote on a large scale, which has caused a large number of missed diagnoses of PCOS. Although the four-indicator model disclosed in CN113035354B has effectively solved some of the problems, the detection of androgens (i.e., androstenedione) in this model is commonly performed in the world by immune-based method in clinical practice. However, due to the inherent technical bottleneck of this technology, the accurate detection of androgen level in a woman by immune-based method has always been recognized as a difficulty in the world. The issue of inaccurate detection of androgen level in a woman by immune-based method has always plagued clinical practice, constraining the diagnosis and treatment of female androgen related diseases. This is also an important reason why the etiology of PCOS is unknown worldwide, and there are no treatment drugs targeting the cause of the disease.

The three-indicator model of this application does not use androgen, avoiding potential misdiagnosis and missed diagnosis opportunities due to inaccurate immune-based androgen testing commonly used in clinical practice. Moreover, research has shown a good correlation between AMH and mass spectrometry based androgen (mass spectrometry androgen testing is more accurate, and is the internationally recognized gold standard for androgen testing) (Zhang X, Xu H, Feng G, et al. Clin Chim Acta doi: 10.1016/j.cca.2022.11.025) PubMed PMID: 36450312.). The weight of AMH in the three-indicator model of this application is the sum of the weights of AMH and androstenedione in the previous four-indicator model, and the model effects of the two models are not different. This indicates that, the new three-indicator model not only saves cost as compared to the previous four-indicator model, but also effectively avoids the problem of inaccurate immune-based androgen detection, which has good economic benefits and practical significance, and therefore has obvious innovation.

The specific embodiments mentioned above are only illustrative and instructive, not restrictive. A person skilled in the art can also make many modifications under the inspiration of this specification without departing from the scope of protection of the claims in this application, all of which belong to the scope of protection in this application.

## Claims

1.   A system for screening polycystic ovary syndrome, comprising:

a data collection module for obtaining anti-Mullerian hormone (AMH) level in a subject, collecting upper limit of the number of menstrual cycle days voluntarily provided by the subject, and collecting BMI of the subject; and a module for calculating a probability of suffering from polycystic ovary syndrome, which is used to calculate the above data information obtained from the data collection module, so as to calculate a probability (p) of a subject suffering from polycystic ovary syndrome.

2.   The system according to claim 1, further comprising:
a grouping module for pre-storing default grouping parameters for polycystic ovary syndrome, grouping the calculated probability (p) of a subject suffering from polycystic ovary syndrome based on the grouping parameters, thereby grouping the risk of a subject suffering from polycystic ovary syndrome.

3. The system according to claim 1 or 2, wherein
in the module for calculating a probability of suffering from polycystic ovary syndrome, the probability (p) of a subject suffering from polycystic ovary syndrome is calculated by converting data of the anti-Mullerian hormone (AMH) level, upper limit of the number of menstrual cycle days, and BMI of the subject into polytomous variables.

4. The system according to claim 1 or 2, wherein
the anti-Mullerian hormone (AMH) level refers to a concentration of AMH in venous blood of a female subject on any day of the menstrual cycle.

5. The system according to claim 1 or 2, wherein

in the module for calculating a probability of suffering from polycystic ovary syndrome, the levels of anti-Mullerian hormone (AMH) are converted into five-categorical variables, that is to say,
the AMH levels are divided into the following five groups: the subjects' AMH levels are less than 2.5 ng/ml, the subjects' AMH levels are 2.5 ng/ml or more but less than 5 ng/ml, the subjects' AMH levels are 5 ng/ml or more but less than 7.5 ng/ml, the subjects' AMH levels are 7.5 ng/ml or more but less than 10 ng/ml, and the subjects' AMH levels are 10 ng/ml or more.

6. The system according to claim 1 or 2, wherein

in the module for calculating a probability of suffering from polycystic ovary syndrome, the upper limits of the number of menstrual cycle days of the subjects are converted into five-categorical variables, that is to say,
the upper limits of the number of menstrual cycle days of the subjects are divided into the following five groups: the upper limits of the number of menstrual cycle days of the subjects are less than 35 days, the upper limits of the number of menstrual cycle days of the subjects are 35 days or more but less than 45 days, the upper limits of the number of menstrual cycle days of the subjects are 45 days or more but less than 60 days, the upper limits of the number of menstrual cycle days of the subjects are 60 days or more but less than 90 days, and the upper limits of the number of menstrual cycle days of the subjects are 90 days or more.

7. The system according to claim 1 or 2, wherein

in the module for calculating a probability of suffering from polycystic ovary syndrome, the BMIs of the subjects are converted into four-categorical variables, that is to say,
the BMIs of the subjects are divided into the following four groups: BMIs of the subjects are less than 18.5, BMIs of the subjects are 18.5 or more but less than 24, BMIs of the subjects are 24 or more but less than 28, and BMIs of the subjects are 28 or more.

8. The system according to claim 1 or 2, wherein
in the module for calculating a probability of suffering from polycystic ovary syndrome, a formula for calculating a probability (p) of suffering from polycystic ovary syndrome is pre-stored, and the formula is obtained by fitting the polytomous variables converted from the data of the anti-Mullerian hormone (AMH) level, upper limit of the number of menstrual cycle days, and BMI of the subject in the existing database.

9. The system according to claim 8, wherein

the above formula is the following Formula I:

$$p = 1/[1 + e^{-(i + a*AMH + b*\text{upper limit of the number of menstrual cycle days} + c*BMI)}] \quad (\text{Formula I})$$

wherein p is the calculated probability of a subject suffering from polycystic ovary syndrome, and a, b, c and i are unitless parameters;
in the module for calculating a probability of suffering from polycystic ovary syndrome, the values of a, b and c are obtained based on the anti-Mullerian hormone (AMH) level, upper limit of the number of menstrual cycle days, and BMI of the subject and are plugged into Formula I for calculation,

and in the calculation, the value of AMH, the upper limit of the number of menstrual cycle days, or BMI is set as 0 or 1.

10. The system according to claim 9, wherein

i is any value selected from -4.255406 to -3.56205;
when the subject's AMH level is less than 2.5 ng/ml, the AMH value is set as 0;
when the subject's AMH level is 2.5 ng/ml or more but less than 5 ng/ml, the AMH value is set as 1, and a is any value selected from 0.3982787 to 0.846642;
when the subject's AMH level is 5 ng/ml or more but less than 7.5 ng/ml, the AMH value is set as 1, and a is any value selected from 1.3775495 to 1.8533111;
when the subject's AMH level is 7.5 ng/ml or more but less than 10 ng/ml, the AMH value is set as 1, and a is any value selected from 2.0095086 to 2.5992375;
when the subject's AMH level is 10 ng/ml or more, the AMH value is set as 1, and a is any value selected from 2.4552789 to 3.0324161;
when the upper limit of the number of menstrual cycle days of the subject is less than 35 days, the value of the upper limit of the number of menstrual cycle days is set as 0;
when the upper limit of the number of menstrual cycle days of the subject is 35 days or more but less than 45 days, the value of the upper limit of the number of menstrual cycle days is set as 1, and b is any value selected from 1.1836336 to 1.6602398;
when the upper limit of the number of menstrual cycle days of the subject is 45 days or more but less than 60 days, the value of the upper limit of the menstrual cycle days is set as 1, and b is any value selected from 1.6734536 to 2.1734828;
when the upper limit of the number of menstrual cycle days of the subject is 60 days or more but less than 90 days, the value of the upper limit of the number of menstrual cycle days is set as 1, and b is any value selected from 1.7119305 to 2.4218832;
when the upper limit of the number of menstrual cycle days of the subject is 90 days or more, the value of the upper limit of the number of menstrual cycle days is set as 1, and b is any value selected from 2.0049859 to 2.682179;
when the subject's BMI is less than 18.5, the BMI value is set as 0;
when the subject's BMI is 18.5 or more but less than 24, the BMI value is set as 1, and c is any value selected from -0.306744 to 0.3390827;
when the subject's BMI is 24 or more but less than 28, the BMI value is set as 1, and c is any value selected from 0.1934385 to 0.8774874; and
when the subject's BMI is 28 or more, the BMI value is set as 1, and c is any value selected from 0.5968209 to 1.3646449.

11. The system according to claim 2, wherein
the grouping basis pre-stored in the grouping module is:

when the calculated probability (p) of a subject suffering from polycystic ovary syndrome is less than 10%, the risk of a subject suffering from polycystic ovary syndrome is low;
when the calculated probability (p) of a subject suffering from polycystic ovary syndrome is 10% or more but less than 50%, the risk of a subject suffering from polycystic ovary syndrome is moderate; and
when the calculated probability (p) of a subject suffering from polycystic ovary syndrome is 50% or more, the risk of a subject suffering from polycystic ovary syndrome is high.

12. A method for screening polycystic ovary syndrome, comprising:

a data collection step for obtaining anti-Mullerian hormone (AMH) level in a subject, collecting upper limit of the number of menstrual cycle days voluntarily provided by the subject, and collecting BMI of the subject; and
a step for calculating a probability of suffering from polycystic ovary syndrome, which is used to calculate the above data information obtained from a data collection module, so as to calculate a probability (p) of a subject suffering from polycystic ovary syndrome.

13. The method according to claim 12, further comprising:
a grouping step for pre-storing default grouping parameters for polycystic ovary syndrome, grouping the calculated probability (p) of a subject suffering from polycystic ovary syndrome based on the grouping parameters, thereby grouping the risk of a subject suffering from polycystic ovary syndrome.

14. The method according to claim 12 or 13, wherein
in the step for calculating a probability of suffering from polycystic ovary syndrome, the probability (p) of a subject suffering from polycystic ovary syndrome is calculated by converting data of the anti-Mullerian hormone (AMH) level, upper limit of the number of menstrual cycle days, and BMI of the subject into polytomous variables.

15. The method according to claim 12 or 13, wherein
the anti-Mullerian hormone (AMH) level refers to a concentration of AMH in venous blood of a female subject on any day of the menstrual cycle.

16. The method according to claim 12 or 13, wherein

in the step for calculating a probability of suffering from polycystic ovary syndrome, the levels of anti-Mullerian hormone (AMH) are converted into five-categorical variables, that is to say,
the AMH levels are divided into the following five groups: the subjects' AMH levels are less than 2.5 ng/ml, the subjects' AMH levels are 2.5 ng/ml or more but less than 5 ng/ml, the subjects' AMH levels are 5 ng/ml or more but less than 7.5 ng/ml, the subjects' AMH levels are 7.5 ng/ml or more but less than 10 ng/ml, and the subjects' AMH levels are 10 ng/ml or more.

17. The method according to claim 12 or 13, wherein

in the step for calculating a probability of suffering from polycystic ovary syndrome, the upper limits of the number of menstrual cycle days of the subjects are converted into five-categorical variables, that is to say,
the upper limits of the number of menstrual cycle days of the subjects are divided into the following five groups: the upper limits of the number of menstrual cycle days of the subjects are less than 35 days, the upper limits of the number of menstrual cycle days of the subjects are 35 days or more but less than 45 days, the upper limits of the number of menstrual cycle days of the subjects are 45 days or more but less than 60 days, the upper limits of the number of menstrual cycle days of the subjects are 60 days or more but less than 90 days, and the upper limits of the number of menstrual cycle days of the subjects are 90 days or more.

18. The method according to claim 12 or 13, wherein

in the step for calculating a probability of suffering from polycystic ovary syndrome, the BMIs of the subjects are converted into four-categorical variables, that is to say,
the BMIs of the subjects are divided into the following four groups: BMIs of the subjects are less than 18.5, BMIs of the subjects are 18.5 or more but less than 24, BMIs of the subjects are 24 or more but less than 28, and BMIs of the subjects are 28 or more.

19. The method according to claim 12 or 13, wherein
in the step for calculating a probability of suffering from polycystic ovary syndrome, a formula for calculating a probability (p) of suffering from polycystic ovary syndrome is pre-stored, and the formula is obtained by fitting the polytomous variables converted from the data of the anti-Mullerian hormone (AMH) level, upper limit of the number of menstrual cycle days, and BMI of the subject in the existing database.

20. The method according to claim 19, wherein

the above formula is the following Formula I:

$$p = 1/[1 + e^{-(i + a*AMH + b*\text{upper limit of the number of menstrual cycle days} + c*BMI)}] \quad (\text{Formula I})$$

wherein p is the calculated probability of a subject suffering from polycystic ovary syndrome, and a, b, c and i are unitless parameters;
in the step for calculating a probability of suffering from polycystic ovary syndrome, the values of a, b and c are obtained based on the anti-Mullerian hormone (AMH) level, upper limit of the number of menstrual cycle days, and BMI of the subject and are plugged into Formula I for calculation,
in the calculation, the value of AMH, the upper limit of the number of menstrual cycle days, or BMI is set as 0 or 1.

21. The method according to claim 20, wherein

i is any value selected from -4.255406 to -3.56205;

when the subject's AMH level is less than 2.5 ng/ml, the AMH value is set as 0;

when the subject's AMH level is 2.5 ng/ml or more but less than 5 ng/ml, the AMH value is set as 1, and a is any value selected from 0.3982787 to 0.846642;

when the subject's AMH level is 5 ng/ml or more but less than 7.5 ng/ml, the AMH value is set as 1, and a is any value selected from 1.3775495 to 1.8533111;

when the subject's AMH level is 7.5 ng/ml or more but less than 10 ng/ml, the AMH value is set as 1, and a is any value selected from 2.0095086 to 2.5992375;

when the subject's AMH level is 10 ng/ml or more, the AMH value is set as 1, and a is any value selected from 2.4552789 to 3.0324161;

when the upper limit of the number of menstrual cycle days of the subject is less than 35 days, the value of the upper limit of the number of menstrual cycle days is set as 0;

when the upper limit of the number of menstrual cycle days of the subject is 35 days or more but less than 45 days, the value of the upper limit of the number of menstrual cycle days is set as 1, and b is any value selected from 1.1836336 to 1.6602398;

when the upper limit of the number of menstrual cycle days of the subject is 45 days or more but less than 60 days, the value of the upper limit of the menstrual cycle days is set as 1, and b is any value selected from 1.6734536 to 2.1734828;

when the upper limit of the number of menstrual cycle days of the subject is 60 days or more but less than 90 days, the value of the upper limit of the number of menstrual cycle days is set as 1, and b is any value selected from 1.7119305 to 2.4218832;

when the upper limit of the number of menstrual cycle days of the subject is 90 days or more, the value of the upper limit of the number of menstrual cycle days is set as 1, and b is any value selected from 2.0049859 to 2.682179;

when the subject's BMI is less than 18.5, the BMI value is set as 0;

when the subject's BMI is 18.5 or more but less than 24, the BMI value is set as 1, and c is any value selected from -0.306744 to 0.3390827;

when the subject's BMI is 24 or more but less than 28, the BMI value is set as 1, and c is any value selected from 0.1934385 to 0.8774874; and

when the subject's BMI is 28 or more, the BMI value is set as 1, and c is any value selected from 0.5968209 to 1.3646449.

22. The method according to claim 13, wherein
the grouping basis pre-stored in the grouping step is:

when the calculated probability (p) of a subject suffering from polycystic ovary syndrome is less than 10%, the risk of a subject suffering from polycystic ovary syndrome is low;

when the calculated probability (p) of a subject suffering from polycystic ovary syndrome is less than 50%, the risk of a subject suffering from polycystic ovary syndrome is moderate; and

when the calculated probability (p) of a subject suffering from polycystic ovary syndrome is 50% or more, the risk of a subject suffering from polycystic ovary syndrome is high.

## INTERNATIONAL SEARCH REPORT

| | International application No. |
|---|---|
| | **PCT/CN2023/075024** |

### A. CLASSIFICATION OF SUBJECT MATTER

G16H 50/20(2018.01)i

According to International Patent Classification (IPC) or to both national classification and IPC

### B. FIELDS SEARCHED

Minimum documentation searched (classification system followed by classification symbols)

IPC: G16H

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 多囊卵巢, 抗缪勒氏管激素, 月经, 概率, BMI, AMH, polycystic ovarian syndrome, POS, menstrua, probability

### C. DOCUMENTS CONSIDERED TO BE RELEVANT

| Category* | Citation of document, with indication, where appropriate, of the relevant passages | Relevant to claim No. |
|---|---|---|
| PX | CN 115620900 A (PEKING UNIVERSITY THIRD HOSPITAL (PEKING UNIVERSITY THIRD CLINICAL MEDICAL COLLEGE) et al.) 17 January 2023 (2023-01-17) description, paragraphs [0006]-[0027] | 1-22 |
| X | CN 113035354 A (PEKING UNIVERSITY THIRD HOSPITAL (PEKING UNIVERSITY THIRD CLINICAL MEDICAL COLLEGE) et al.) 25 June 2021 (2021-06-25) description, paragraphs [0006]-[0029] | 1-8, 11-19, 22 |
| Y | CN 113035354 A (PEKING UNIVERSITY THIRD HOSPITAL (PEKING UNIVERSITY THIRD CLINICAL MEDICAL COLLEGE) et al.) 25 June 2021 (2021-06-25) description, paragraphs [0006]-[0029] | 9-10, 20-21 |
| Y | 帕孜力亚 牙生 等 (PAZILIYA, Yasheng et al.). "育龄期多囊卵巢综合征风险预测模型的构建 (A Nomogram Model for Predicting Polycystic Ovary Syndrome)" 新疆医科大学学报 (Journal of Xinjiang Medical University), Vol. 43, No. 08, 15 August 2020 (2020-08-15), pages 1113-1117 and 1121, and sections 2 and 3 | 9-10, 20-21 |
| A | CN 113049838 A (SHANDONG UNIVERSITY) 29 June 2021 (2021-06-29) entire document | 1-22 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **03 June 2023** | **08 June 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/075024** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2022044822 A1 (GUANGZHOU KANGRUN BIOTECH CO., LTD.) 10 February 2022 (2022-02-10)<br>    entire document | 1-22 |

Form PCT/ISA/210 (second sheet) (July 2022)

**INTERNATIONAL SEARCH REPORT**
Information on patent family members

International application No.

**PCT/CN2023/075024**

| Patent document cited in search report | | | Publication date (day/month/year) | Patent family member(s) | | | Publication date (day/month/year) |
|---|---|---|---|---|---|---|---|
| CN | 115620900 | A | 17 January 2023 | None | | | |
| CN | 113035354 | A | 25 June 2021 | WO | 2022246882 | A1 | 01 December 2022 |
| CN | 113049838 | A | 29 June 2021 | None | | | |
| US | 2022044822 | A1 | 10 February 2022 | EP | 3896453 | A1 | 20 October 2021 |
| | | | | WO | 2020118790 | A1 | 18 June 2020 |
| | | | | CN | 109602394 | A | 12 April 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 113035354 B

**Non-patent literature cited in the description**

- **ZHANG X** ; **XU H** ; **FENG G et al.** *Clin Chim Acta*

- Revised 2003 concerns on diagnostic criteria and long-term health risks related to polycystic ovary syndrome.. *Fertil Steril*, 2004, vol. 81, 19-25